# EUROPÄISCHE PATENTSCHRIFT

(11) **EP 3 593 910 B1**
(45) Veröffentlichungstag und Bekanntmachung des Hinweises auf die Patenterteilung: **15.01.2025**
(21) Anmeldenummer: 19184374.7
(22) Anmeldetag: 04.07.2019
(51) Int. Cl.: B05D 3/14, H01J 37/32, B05D 3/04, B05D 7/22, H05H 1/24

(54) **VERFAHREN ZUR BESCHICHTUNG EINES GLASSPRITZENKÖRPERS FÜR EINE HYPODERMISCHE FERTIGGLASSPRITZE, HYPODERMISCHE FERTIGGLASSPRITZE SOWIE PLASMABEHANDLUNGSVORRICHTUNG FÜR GLASSPRITZENKÖRPER HYPODERMISCHER FERTIGGLASSPRITZEN**
METHOD FOR COATING A GLASS SYRINGE BODY FOR A HYPODERMIC READY-FOR-USE GLASS SYRINGE, HYPODERMIC READY-FOR-USE GLASS SYRINGE AND PLASMA TREATMENT DEVICE FOR GLASS SYRINGE BODIES OF HYPODERMISCHER READY-FOR-USE GLASS SYRINGES
PROCÉDÉ DE REVÊTEMENT D'UN CORPS DE FORMAGE PAR INJECTION POUR UN SERINGUE HYPODERMIQUE DE VERRE FINI, SERINGUE HYPODERMIQUE DE VERRE FINI AINSI QUE DISPOSITIF DE TRAITEMENT AU PLASMA POUR CORPS DE FORMAGE PAR INJECTION DE LA SERINGUE HYPODERMIQUE DE VERRE FINI

(30) Priorität: 09.07.2018 DE 102018116560
(43) Veröffentlichungstag der Anmeldung: 15.01.2020
(73) Patentinhaber: Gerresheimer Regensburg GmbH, 92442 Wackersdorf (DE)
(72) Erfinder: Daniel, Christian, 92533 Wernberg Köblitz (DE); Baier, Lukas, 93049 Regensburg (DE)
(74) Vertreter: Schmid, Nils T.F.

(56) Entgegenhaltungen:
- WO-A1-2007/137890
- WO-A1-2011/029628
- WO-A1-2013/170044
- WO-A1-2019/007876
- DE-A1- 102011 009 057

## Beschreibung

Die vorliegende Erfindung betrifft ein Verfahren zur Beschichtung eines Glasspritzenkörpers für eine hypodermische Fertigglasspritze. Ferner betrifft die vorliegende Erfindung eine hypodermische Fertigglasspritze. Des Weiteren betrifft die vorliegende Erfindung auch eine Plasmabehandlungsvorrichtung für Glasspritzenkörper hypodermischer Fertigglasspritzen.

Hypodermische Fertigglasspritzen sind im Allgemeinen zur Anwendung an der Haut eines Patienten vorgesehen, insbesondere dazu, zu dessen Unterhaut vorzudringen. Derartige gattungsgemäße Spritzen umfassen in der Regel eine längliche, zylindrische Hülse mit zwei sich diametral gegenüberliegenden Enden und einer Kammer, die sich zwischen den beiden Enden ausbildet, zum Aufbewahren einer Substanz, beispielsweise eines Fluids vorzugsweise mit einem Wirkstoff. Das eine der beiden Enden besitzt eine Öffnung, um das Einführen eines Kolbens zu gewährleisten, und gegebenenfalls einen Flansch zum Betätigen mit den Fingern durch einen Benutzer. Das andere der beiden Enden verjüngt in der Regel im Querschnitt zur Bildung eines Spritzenkonus und ist im Wesentlichen geschlossen bis auf einen engen Durchgang bzw. Endkanal, der sich von der Hülsenkammer durch den Spritzenkonus hindurch erstreckt und das Austreten bzw. Ausschieben der Substanz aus der Kammer durch den Spritzenkonus ermöglicht. Eine Kanüle kann an dem Spritzenkonus-Ende angebracht werden, wobei beispielsweise die Kanüle innerhalb des Durchgangs mittels eines Klebstoffes befestigbar ist. Alternativ kann die Kanüle auch von außen an den Hülsenkörper mittels eines Kanülenhalters befestigt werden. Dabei ist als Kanüle allgemein eine hohle Nadel zu verstehen, die dazu benutzt wird, in menschliches oder tierisches Gewebe einzudringen, um mit Hilfe der Spritze das Fluid zu injizieren oder Körperflüssigkeiten zu entnehmen

Es ist bekannt, eine Glasspritze mit einer Gleitmittelschicht, wie einer Silikonschicht, innenseitig zu versehen, um zum einen eine Reduzierung der Reibung zwischen Kolben und Hülsenkörperinnenfläche zum leichteren Bewegen des Kolbens relativ zu dem Hülsenkörper zu erreichen und zum anderen eine vollumlaufende Dichtungskontaktfläche einfach zwischen der harten Glaskörperinnenseite und der harten Kolbenaußenseite aufgrund der Deformierbarkeit der Gleitmittelschicht zu gewährleisten. Grundsätzlich bestehen dabei zwei Möglichkeiten. Zum einen kann die Glasspritze mit einer Gleitmittelschicht im Inneren beschichtet werden, wobei zum Beispiel öliges Silikon zum Einsatz kommt. Hierbei wird die Kanüle vor der Beschichtung mit der Gleitmittelschicht eingeklebt. Eine weitere Möglichkeit stellt die Einbrennsilikonisierung dar, die insbesondere dann angewendet wird, falls eine härtere Haftung des Silikonöls an der Spritze erforderlich ist. In diesem Zusammenhang ist Einbrennen als Härten zu verstehen. Bei dieser Technik ist es allerdings nicht möglich, die Kanüle vor der Silikonisierung einzuleben, da die Kanüle den hohen Temperaturen, die bei der Einbrennsilikonisierung auftreten, nicht standhält. Somit besteht das bisher auf dem Gebiet der mit einer Gleitmittelinnenschicht versehenen Glasspritzen, insbesondere bei einbrennsilikonisierten Glasspritzen, ungelöste Problem, in dem Endkanal der Glasspritze eine Kanüle bei ausreichenden Adhäsions- und Festigkeitseigenschaften zu verkleben. Es zeigte sich, dass keine ausreichende Adhäsion zwischen Klebstoff und Glas aufgrund des Vorhandenseins der Silikonschicht aufgebaut werden kann.

Eine Lösung für dieses Problem besteht darin, eine Silikonschicht anklebefähig zu machen, indem ein Atmosphärenplasma zur Vorbehandlung der Klebemittelschicht eingesetzt wird. Allerdings ist es nicht möglich, insbesondere aufgrund der dimensionalen Eigenart der Glasspritze vor allem im Bereich des endseitigen Innenkanals einen vollständigen Zugriff des Plasmas auf die Gleitmittelschicht zu bewirken, um somit eine zuverlässige Haftung der Kanüle in dem Endkanal zu realisieren.

Aus EP 1 352 667 ist beispielsweise ein Verfahren zum Herstellen einer Injektionsspritze bekannt, bei dem ein Spritzenkörper bei hoher Temperatur im Inneren mit einem Gleitmittel versehen wird. An der Fügestelle zwischen Spritzenkörper und Kanüle wird das Gleitmittel darauffolgend beispielsweise mittels Plasmabehandlung entfernt, um eine Haftung des zum Fügen verwendeten Klebstoffes an der Spritzenkörperinnenfläche und damit ein Haften der Kanüle an dem Spritzenkörper zu ermöglichen. Dieses bekannte Verfahren weist den Nachteil auf, dass das vollständige Entfernen des Gleitmittels in dem räumlich eng begrenzten Bereich an der Spitze des Hülsenkörpers nur mit großem Aufwand erreicht werden kann.

WO 2011/029628 A1 offenbart ein pharmazeutisches Packmittel mit einem Gleitfilm und ein Verfahren zu dessen Herstellung. Ferner offenbart WO 2007137890 A1 ein Verfahren und eine Vorrichtung zur Oberflächenbehandlung an Behältnissen oder Gegenständen.

Es ist daher Aufgabe der Erfindung, die Nachteile aus dem bekannten Stand der Technik zu überwinden, insbesondere ein Verfahren zur Beschichtung eines mit einer Gleitmittelinnenschicht versehenen, insbesondere silikonisierten oder einbrennsilikonisierten, Glasspritzenkörpers für eine hypodermische Fertigglasspritze bereitzustellen, bei dem die Haltekraft zwischen Kanüle und Glasspritzenkörper erhöht ist, insbesondere um Haltekräfte gemäß DIN ISO 7864 zu erzielen, und bei dem der schmale Endkanal der Glasspritze einer verbesserten Behandlung mit Atmosphärenplasma unterzogen werden kann. Es ist ferner Aufgabe der vorliegenden Erfindung, eine hypodermische Fertigglasspritze bereitzustellen, die eine erhöhte Haltekraft zwischen Glasspritze und Kanüle aufweist und verbesserten Behandlung mit Atmosphärenplasma unterzogen ist. Des Weiteren besteht eine Aufgabe der vorliegenden Erfindung darin, eine Plasmabehandlungsvorrichtung für einen Glasspritzenkörper einer hypodermischen Fertigglasspritze bereitzustellen, mit der eine erhöhte Haltekraft zwischen Kanüle und Glasspritzenkörper erzielbar ist und eine verbesserte Behandlung mit Atmosphärenplasma vollzogen werden kann.

Diese Aufgabe wird durch die Merkmale der unabhängigen Ansprüche gelöst.

Danach wird ein Verfahren zur Beschichtung eines Glasspritzenkörpers für eine hypodermische Fertigglasspritze vorgeschlagen, bei welchem zumindest auf eine Innenfläche der hypodermischen Fertigglasspritze zumindest eine Emulsion und/oder eine Lösung aufgebracht wird, welche zumindest eine schichtbildende Substanz enthält. Die Fertigglasspritze definiert eine Axialrichtung, welche im Wesentlichen parallel zu einer Längserstreckungsrichtung der Fertigglasspritze verläuft. Die hypodermische Fertigglasspritze kann eine ebene oder eine gekrümmte Innenfläche aufweisen. Insbesondere kann die hypodermische Glasspritze zunächst ein Halbzeug sein, welches in nachfolgenden Verfahrensschritten zu einem Fertigprodukt weiterbearbeitet wird. In anderen Ausführungsformen der Erfindung kann die hypodermische Glasspritze auch als Fertigprodukt ausgebildet sein, welches nach Durchführung des vorgeschlagenen Beschichtungsverfahrens keiner weiteren Bearbeitung mehr bedarf.

Die Beschichtung enthält zumindest eine schichtbildende Substanz, welche in Form einer Emulsion und/oder einer Lösung vorliegt. Die Emulsion oder die Lösung enthält neben der schichtbildenden Substanz zumindest ein Lösemittel. Für die Zwecke der vorliegenden Beschreibung wird die Trägermatrix einer Emulsion ebenfalls als Lösemittel bezeichnet, auch wenn die schichtbildenden Substanzen nur emulgiert und nicht im strengeren Sinne gelöst sind.

Die Emulsion oder die Lösung kann durch an sich bekannte Verfahren auf zumindest eine Innenfläche der hypodermischen Fertigglasspritze aufgebracht werden. Beispielsweise kann auch eine Vollbeschichtung der hypodermischen Fertigglasspritze vorgesehen sein, d.h. die gesamte Innenfläche der hypodermischen Fertigglasspritze wird beschichtet. Die Emulsion oder die Lösung kann beispielsweise durch Sprühen, Drucken, Streichen oder Tauchen aufgebracht werden. Um eine gleichmäßigere Beschichtung mit der Emulsion bzw. der Lösung zu ermöglichen, können zusätzliche Maßnahmen ergriffen werden, beispielsweise das Herabsetzen der Viskosität durch Erwärmen und/oder Verdünnen oder eine Unterstützung der Beschichtung durch elektrische Felder, wie bei einer Elektrotauchlackierung.

Die hypodermische Fertigglasspritze kann nach dem Aufbringen der Emulsion bzw. der Lösung wärmebehandelt werden. Die Wärmebehandlung kann unmittelbar nach dem Aufbringen der Emulsion bzw. der Lösung erfolgen. Beispielsweise kann die Wärmebehandlung nach einem vorangegangenen Trocknungsschritt durchgeführt werden. Die Wärmebehandlung führt einerseits dazu, dass das Lösungsmittel der Emulsion bzw. der Lösung verdampft bzw. aus der Schicht ausgetrieben wird. Darüber hinaus kann eine Verbindung zwischen den schichtbildenden Substanzen und der Innenfläche der hypodermischen Fertigglasspritze entstehen, beispielsweise durch kovalente Bindungen oder Van-der-Waals-Bindungen, die eine Härtung der Schicht bewirken. In einigen Ausführungsformen der Erfindung können die schichtbildenden Substanzen auch untereinander vernetzt sein und beispielsweise ein Polymer oder eine polymerartige Verbindung ausbilden. In einer beispielhaften Ausführung der Erfindung ist die auf diese Weise erzeugte Beschichtung hydrophob und/oder weist gegenüber der unbehandelten Oberfläche verbesserte Gleiteigenschaften auf. Vorzugsweise kann die erzeugte Beschichtung auch chemisch inert gegenüber Gasen oder Flüssigkeiten sein, mit welchen die hypodermische Fertigglasspritze bei bestimmungsgemäßem Gebrauch in Kontakt kommt.

Erfindungsgemäß wird in einem nachfolgenden Verfahrensschritt zumindest eine Teilfläche der mit der Beschichtung versehenen Innenfläche der hypodermischen Fertigglasspritze einer Plasmabehandlung unterzogen. Beispielsweise handelt es sich bei der Teilfläche um eine Innenfläche in einem Spritzenkonus der Fertigglasspritze. Dadurch können die Wirkungen der Beschichtung zumindest teilweise deaktiviert bzw. rückgängig gemacht werden, ohne die Beschichtung als solche vollständig zu entfernen. Unter einem Plasma im Sinne der vorliegenden Beschreibung wird ein teilweise ionisiertes Gas verstanden, welches bei einem vorgebbaren Druck und mit vorgebbarer Zusammensetzung für eine bestimmte Zeitdauer auf zumindest eine Teilfläche der Beschichtung einwirkt. In einer beispielhaften Ausführungsform der Erfindung kann die Teilfläche ebenso groß sein wie die beschichtete Innenfläche, wodurch das Plasma auf die gesamte vorher aufgebrachte Beschichtung auf der Innenfläche einwirkt. Sofern die beschichtete Innenfläche die gesamte Innenfläche der hypodermischen Fertigglasspritze umfasst, kann das Plasma auch auf die gesamte Innenfläche der hypodermische Fertigglasspritze einwirken. Gemäß einer weiteren beispielhaften Ausführung der Erfindung wird nur ein Teil der Beschichtung der Plasmabehandlung unterworfen, wohingegen wenigstens ein weiterer Teil der Beschichtung nicht dem Plasma ausgesetzt wird. Beispielsweise können diejenigen Teilflächen der Plasmabehandlung unterzogen werden, wie beispielsweise die Teilfläche in dem Spritzenkonus der Fertigglasspritze, welche in einem nachfolgenden Verfahrensschritt eine Fügestelle ausbilden sollen, um auf diese Weise die Haftung von Klebstoffen oder Lot zu verbessern oder überhaupt erst zu ermöglichen. Das Plasma wird beispielsweise durch eine Atmosphärendruckplasmaquelle generiert, welche in Axialrichtung gesehen vor einem Ende des Spritzenkonus positioniert wird, um das Plasma über einen in dem Spritzenkonus ausgebildeten Endkanal bzw. Durchgang in das Spritzeninnere einzubringen.

Relativ zu dem Spritzenkonus in Axialrichtung gegenüberliegend zu der Atmosphärendruckplasmaquelle wird eine Unterdruckquelle angeordnet, wobei mittels der Unterdruckquelle ein Unterdruck kleiner als Atmosphärendruck in dem Spritzeninneren, vorzugsweise in dem Spritzenkonusinneren, insbesondere in einem Ausschnitt des Spritzenkonusinneren, bereitgestellt wird. Gemäß einer beispielhaften Ausführung wird als Unterdruck etwa ein Vakuum angelegt. Das Anlegen von Unterdruck im Spritzeninneren ermöglicht das Eindringen des Plasma als solches sowie eine Steigerung der Eindringtiefe des Plasma in den Spritzenkonus, wodurch eine deutlich größere Fläche, insbesondere welche nachfolgend als Klebefläche für die Kanüle bzw. Injektionsnadel verwendet wird, mittels der Atmosphärendruckplasmaquelle behandelbar wird. Durch die Vergrößerung der durch Plasma behandelten Fläche können die Haltekräfte zwischen Kanüle und Glasspritze deutlich vergrößert werden, wobei insbesondere die Adhäsion zwischen Klebstoff und Glas deutlich erhöht wird, wodurch es vorzugsweise ermöglicht ist, Haltekräfte gemäß DIN ISO 7864 zu erzielen. Ferner bewirkt das Anordnen der Unterdruckwelle zum Bereitstellen von Unterdruck im Spritzeninneren eine vorteilhafte Oberflächenaktivierung für das nachträgliche Kleben. Des Weiteren können auch andere unerwünschte Partikel, wie Staub oder Schmutz, aus dem Spritzeninneren entfernt werden, um eine möglichst saubere, d. h. fremdpartikelfreie, Oberfläche zu erhalten. Dadurch kann wiederum eine bessere Adhäsion des Klebstoffs an der Glaskörperinnenfläche erzielt werden.

Gemäß einer bevorzugten Ausführungsform der vorliegenden Erfindung wird ein Saugdorn der Unterdruckquelle in das Glasspritzenkörperinnere, vorzugsweise in einen Kolbenraum des Glasspritzenkörpers, eingeführt. Als Kolbenraum ist dasjenige Volumen zu verstehen, welches von außen her durch die Glasspritze selbst begrenzt ist und in das ein Kolben zum Ausschieben von in dem Kolbenraum angeordneter Substanz, wie einer Flüssigkeit vorzugsweise mit einem Wirkstoff, von außen her, insbesondere in Axialrichtung von der der Atmosphärendruckplasmaquelle gegenüberliegenden Seite der Glasspritze, einschiebbar ist. Über diesen Saugdorn, welcher von der Dimensionierung an einen Innendurchmesser des Glasspritzenkörpers angepasst ist, lässt sich gezielt und je nach Anforderung ein Unterdruck gewünschter Stärke in dem Glaskörperinneren, vorzugsweise in dem Spritzenkonus, vorzugsweise in einem Ausschnitt des Spritzenkonus, erzeugen.

Gemäß einer Weiterbildung der vorliegenden Erfindung wird der Saugdorn in einen vorzugsweise abdichteten Berührkontakt mit einer Innenseite und/oder einer Bodenfläche des Kolbenraums gebracht. Beispielsweise kann der Saugdorn derart bezüglich des Innendurchmessers der Glasspritze, insbesondere des Kolbenraums der Glasspritze, dimensioniert sein, dass ein vollumfänglicher Berührkontakt zwischen Saugdorn und Glasspritzeninnenfläche in Axialrichtung gesehen wenigstens teilweise besteht. Alternativ oder zusätzlich ist denkbar, Dichtmittel, wie einen Dichtring oder einen Dichtungsschlauch, zum Herstellen des abdichteten Berührkontakts zwischen Glasspritzeninnenfläche und Saugdorn vorzusehen. Vorzugsweise befindet sich das Dichtmittel, insbesondere der Dichtring, an einem der Atmosphärendruckplasmaquelle zugewandten Stirnende des Saugdorns.

In einer beispielhaften Ausführung der Erfindung wird durch die Einwirkung des Plasmas der Kohlenstoffgehalt der Beschichtung auf weniger als etwa 80% oder weniger als etwa 75% oder weniger als etwa 70% oder weniger als etwa 60% des Ausgangswertes vor der Plasmabehandlung reduziert. Die Reduktion des Kohlenstoffgehaltes kann darüber hinaus auch die Wirkung haben, dass sich die Bindungen innerhalb der Beschichtung ändern. Beispielsweise kann die Beschichtung von einer polymeren Struktur in eine glasartige oder amorphe Struktur umgewandelt werden. Alternativ oder zusätzlich kann durch die Einwirkung des Plasmas die Schichtdicke in der behandelten Teilfläche abnehmen. In einer weiteren beispielhaften Ausführung der Erfindung kann die Schichtdicke um mehr als etwa 20% oder mehr als etwa 25 % oder mehr als etwa 30 % reduziert werden. Es wurde herausgefunden, dass die Beschichtung durch die Einwirkung des Plasmas nicht vollständig entfernt wird bzw. entfernt werden braucht. Vielmehr kann nach der Plasmabehandlung in der Teilfläche eine Schichtdicke von mehr als etwa 70% oder mehr als etwa 60 % oder mehr als etwa 50 % der im ersten Verfahrensschritt aufgebrachten Schichtdicke verbleiben.

Ferner wurde erkannt, dass durch die Plasmabehandlung die Bindungsverhältnisse und/oder die Schichtdicke und/oder das Elementinventar der ursprünglich aufgebrachten Beschichtung in der dem Plasma ausgesetzten Teilfläche verändert werden kann. Beispielsweise kann eine Aufrauung der Beschichtung und/oder eine Hydrophilierung erzielt werden, sodass ein nachfolgendes Beschichtungs- oder Fügeverfahren erleichtert oder überhaupt erst möglich gemacht werden kann. Des Weiteren kann die Plasmabehandlung einfacher durchzuführen sein als eine vollständige Entfernung der Beschichtung, welche unter Umständen sehr stark an der Innenfläche der hypodermischen Fertigglasspritze haftet und nur mechanisch oder nasschemisch entfernt werden kann, wobei einerseits die entstehende Umweltbelastung erfindungsgemäß vermieden werden kann und andererseits aufwendige Maßnahmen zur Beschränkung des Zutritts eines flüssigen oder gasförmigen Ätzmediums außerhalb der zu behandelnden Teilfläche vermieden werden kann.

In einer beispielhaften Ausführung der Erfindung beträgt die Schichtdicke der Beschichtung zumindest in der Teilfläche vor der Einwirkung des Plasmas zwischen etwa 20 nm und etwa 100 nm oder zwischen etwa 30 nm und etwa 70 nm. Eine solche Beschichtung beeinträchtigt die Maßhaltigkeit der beschichteten Innenfläche der hypodermischen Fertigglasspritze nicht, kann jedoch bereits ausreichen, um verbesserte Gleiteigenschaften, eine Hydrophobierung und/oder eine Passivierung der Oberfläche zu bewirken.

Gemäß einer Weiterbildung der Erfindung kann die Emulsion und/oder die Lösung zumindest ein Silikonöl und ein Lösungsmittel enthalten oder daraus bestehen beispielsweise kann das Lösungsmittel Wasser sein oder Wasser enthalten oder aliphatische Kohlenwasserstoffe oder Aromaten enthalten, beispielsweise Hexan, Heptan, Toluol und/oder Xylol. Schließlich ist es auch denkbar, dass das Lösungsmittel zur Bildung einer Emulsion auch einen Alkohol und/oder Glyzerin und/oder Äther enthält. Eine weitere beispielhafte Ausführung des Lösungsmittels besteht darin, dass es entweder bei normalen Umgebungsbedingungen oder bei erhöhter Temperatur im Wärmeschrank oder Ofen verdampft bzw. aus der entstehenden Beschichtung ausgetrieben wird, sodass eine Beschichtung auf der Innenfläche bzw. auf einer Teilfläche der beschichteten Innenfläche, der hypodermischen Fertigglasspritze verbleibt, welche Silikon enthält oder daraus besteht.

Die Beschichtung kann in einer beispielhaften Ausführung zumindest Kohlenstoff und Sauerstoff und Wasserstoff und Silizium enthalten. Ferner kann die Beschichtung zumindest ein Poly-organo-siloxan enthalten oder daraus bestehen. Durch die Einwirkung des Plasmas kann der Kohlenstoff aus der Beschichtung zumindest teilweise entfernt werden, sodass die Beschichtung nach der Plasmabehandlung Siliziumoxid und/oder Siliziumnitrid und/oder Siliziumoxinitrid enthält oder daraus besteht.

In einer weiteren beispielhaften Ausführung ist die Teilfläche der Innenfläche vor der Plasmabehandlung hydrophob kann die Teilfläche der Innenfläche vor der Plasmabehandlung hydrophil und nach der Plasmabehandlung hydrophob sein. Unter einer hydrophoben Beschichtung wird für die Zwecke der vorliegenden Beschreibung eine Oberfläche verstanden, welche bei Kontakt mit Wasser einen Kontaktwinkel von mehr als 90° ausbildet. Eine hydrophile Oberfläche ist für die Zwecke der vorliegenden Beschreibung eine Oberfläche, welche bei Benetzung mit Wasser einen Kontaktwinkel von weniger als 90° ausbildet. Durch die Plasmabehandlung kann die Oberflächenenergie daher soweit geändert werden, dass die Haftfestigkeit bzw. die Benetzbarkeit mit einem Schmiermittel, einem Klebstoff, einem Lot oder einem Lack ansteigt bzw. überhaupt erst ermöglicht wird.

Das Plasma kann beispielsweise ein Aktivgas enthalten oder daraus bestehen. Vorzugsweise enthält das Aktivgas beispielsweise Stickstoff und/oder Sauerstoff oder synthetische Luft oder atmosphärische Luft, oder besteht daraus.

In einer weiteren beispielhaften Ausführung der Erfindung kann das Plasma für etwa 0,4 bis etwa 5 Sekunden oder für etwa 0,5 bis etwa 4 Sekunden oder für etwa 0,5 bis etwa 1,5 Sekunden oder für etwa 5 bis etwa 60 Sekunden einwirken. Es wurde erkannt, dass diese kurze Behandlungsdauer bereits ausreicht, um eine hydrophobe Silikonbeschichtung in eine hydrophile, siliziumhaltige Verbindung umzusetzen, sodass das Verfahren gemäß der vorliegenden Erfindung auch bei der Herstellung von Massenprodukten wirtschaftlich eingesetzt werden kann.

In einer Weiterbildung der Erfindung kann ein Atmosphärendruckplasma zum Einsatz kommen, welches mit einer dielektrisch behinderten Entladung erzeugt wird. Auf diese Weise lässt sich das Verfahren leicht in bestehende Produktionsprozesse integrieren. Zum einen hängt dies damit zusammen, dass die Verwendung einer dielektrisch behinderten Entladung dafür sorgt, dass die in das Plasma eingekoppelte elektrische Leistung begrenzt bleibt. Zum anderen können nämlich thermische Schäden und/oder der Beschichtung der hypodermischen Fertigglasspritze vermieden werden.

In einer beispielhaften Ausführung der Erfindung kann zur Erzeugung des Plasmas eine Wirbelströmung des Arbeitsgases zum Einsatz kommen. Dadurch kann die Entladung in die Länge gezogen werden. Insbesondere die Behandlung der Innenflächen zylindrischer Hohlkörper verstärkt diesen Effekt noch, da die dort vorhandenen Oberflächen die Entladung unterstützen. Schlanke Gegenstände, wie Spritzen, insbesondere der Spritzenkonus, sind somit auf einfachere Weise zu behandeln bzw. überhaupt erst behandelbar.

In einer beispielhaften Ausführung der Erfindung kann das Plasma ein Inertgas enthalten oder daraus bestehen. Vorzugsweise kann das Inertgas ein Edelgas sein oder ein solches enthalten. Es ist auch möglich, dass das Plasma Argon enthält oder daraus besteht. Ein Inertgasplasma sorgt insbesondere dafür, dass die Bestandteile der Beschichtung nicht oxidiert oder reduziert werden oder anderweitig mit dem Prozessgas des Plasmas reagieren, so dass sich die Beschichtung nicht in unerwünschter Weise umsetzt oder durch reaktives Ätzen von der Oberfläche entfernt wird.

In einer Weiterbildung der vorliegenden Erfindung kann das Plasma durch eine Wechselspannung oder eine gepulste Spannung erzeugt werden, welche eine Frequenz zwischen etwa 10 kHz und etwa 30 kHz oder zwischen etwa 15 kHz und etwa 25 kHz aufweist.

Gemäß einer beispielhaften Ausführung der Erfindung kann ein Plasmastrahl verwendet werden, welcher durch ein elektrisches Feld zwischen der Oberfläche des Bauteils und zumindest einer Gegenelektrode durch Ionisieren eines Arbeitsgasstromes erzeugt wird. Es ist außerdem möglich, ein Plasmajet zu verwenden, wobei das Plasma im Inneren der Plasmaquelle durch elektrische Felder oder elektromagnetische Strahlung erzeugt und vom Arbeitsgasstrom aus der Quelle ausgetrieben wird. In beispielhaften Ausführungsformen der Erfindung wird das Plasma mit einer dielektrisch behinderten Entladung erzeugt. Solchermaßen erzeugte Plasmen weisen nur geringe Temperaturerhöhungen von weniger als etwa 50 K oder weniger als etwa 30 K gegenüber der Umgebung auf, sodass thermische Schäden auch bei empfindlichen Oberflächen vermieden werden können. Dies ist beispielsweise bei der Behandlung von Bauteilen aus einem Polymer oder anderen Kunststoffen hilfreich.

Bei einer Weiterbildung der vorliegenden Erfindung wird das Plasma als Plasmastrahl oder Plasmajet erzeugt, welcher zumindest auf die Teilfläche der Innenfläche des Spritzenkonus einwirkt. Des Weiteren kann vorgesehen sein, dass das Plasma insbesondere nur dann einwirkt, wenn der Unterdruck durch die Unterdruckquelle bereitgestellt wird.

Gemäß einem weiteren Aspekt der vorliegenden Erfindung, der mit den vorigen Aspekten kombinierbar ist, wird bei einem Verfahren zur Herstellung einer hypodermischen Fertigglasspritze mit einem Glasspritzenkörper, der gemäß dem erfindungsgemäßen, oben beschriebenen, Beschichtungsverfahren beschichtet wird, und einer Injektionsnadel, welche miteinander bzw. aneinander gefügt werden, die Injektionsnadel entlang einer Fügestelle mit dem Glasspritzenkörper verbunden. Die Fügestelle kann dabei die Teilfläche in dem Spritzenkonus der Fertigglasspritze umfassen. Das erfindungsgemäße Beschichtungs- bzw. Behandlungsverfahren durch Anordnen einer Atmosphärendruckplasmaquelle und einer Unterdruckquelle an der Fertigglasspritze derart, dass die Teilfläche, die von der Fügestelle umfasst ist, vor dem Aneinanderfügen von Injektionsnadel und Glasspritzenkörper beschichtet bzw. behandelt wird, bewirkt die erfindungsgemäßen, oben beschriebenen Vorteile der Fertigglasspritze, insbesondere erhöhte Nadelhaltekräfte in Spritzenkörper-Axialrichtung und/oder insbesondere eine verstärkte Adhäsion des Klebstoffes an dem Glasspritzenkörper.

In einer Weiterbildung der vorliegenden Erfindung kann das Fügen unter Verwendung eines Klebstoffes erfolgen, welcher insbesondere ausgewählt ist aus einem Acrylat und/oder Polyurethan und/oder einem Epoxidharz und/oder einem Cyanacrylat. Solche Klebstoffe weisen hohe Haftfestigkeiten bei einfacher Verarbeitbarkeit und hohen Anfangsfestigkeiten auf, sodass eine rasche Produktion ermöglicht ist.

Gemäß einem weiteren Aspekt der vorliegenden Erfindung, der mit den vorherigen Aspekten kombinierbar ist, umfasst eine hypodermische Fertigglasspritze einen Glasspritzenkörper und eine Injektionsnadel, die entlang einer Fügestelle durch eine Klebstoffschicht miteinander verbunden sind. Die Fügestelle umfasst vorzugsweise zumindest eine Teilfläche einer Innenfläche eines Spritzenkonus des Glasspritzenkörpers. Die Klebstoffschicht zwischen Fügestelle und Injektionsnadel stellt eine Nadelhaltekraft in Axialrichtung bereit, um zu gewährleisten, dass die Injektionsnadel an der Fügestelle während einer Anwendung der Fertigglasspritze haften bleibt. Erfindungsgemäß ist auf die Innenfläche des Spritzenkonus zumindest eine atmosphärenplasmabehandelte Beschichtung aufgebracht. Die Nadelhaltekraft beträgt dabei vorzugsweise wenigstens
(a) 11 N für Nadeldurchmesser kleiner als 0,33 mm,
(b) 22 N für Nadeldurchmesser kleiner als 0,55 mm,
(c) 34 N für Nadeldurchmesser kleiner als 0,7 mm,
(d) 40 N für Nadeldurchmesser kleiner als 0,8 mm,
(e) 44 N für Nadeldurchmesser kleiner als o,9 mm,
(f) 54 N für Nadeldurchmesser kleiner als 1,1 mm, und/oder
(g) 44 N für Nadeldurchmesser größer oder gleich 1,1 mm.

Die dabei erzielten Haltekräfte sind ausreichend, um die Festigkeitserfordernisse gemäß DIN ISO 7864 zu gewährleisten und damit eine ausreichende Adhäsion zwischen Injektionsnadel und Glasspritzenkörper zu erzielen.

Gemäß einer beispielhaften Ausführung der Erfindung beträgt die Schichtdicke der Beschichtung zumindest in der Teilfläche der Innenfläche vor der Einwirkung des Plasmas zwischen etwa 20 nm und etwa 100 nm oder zwischen etwa 30 nm und etwa 70 nm. Durch die Plasmabehandlung in der Teilfläche der Innenfläche kann die Schichtdicke der Beschichtung um mehr als etwa 20 % oder mehr als etwa 25 % oder mehr als etwa 30 % abnehmen. Alternativ oder zusätzlich kann nach der Plasmabehandlung der Teilfläche eine Schichtdicke von mehr als etwa 70 % oder mehr als etwa 60 % oder mehr als etwa 50 % verbleiben.

Gemäß einem weiteren Aspekt der vorliegenden Erfindung kann die Beschichtung zumindest Kohlenstoff und Sauerstoff und Wasserstoff und Silicium enthalten. Alternativ oder zusätzlich kann die Beschichtung zumindest ein Poly(organo)siloxan enthalten. Vorzugsweise ist der Kohlenstoffgehalt der Beschichtung auf weniger als etwa 80 % oder weniger als etwa 75 % oder weniger als etwa 70 % oder weniger als etwa 60 % des Ausgangswertes vor der Plasma Behandlung reduziert.

In einer weiteren beispielhaften Ausführung der erfindungsgemäßen Fertigglasspritze ist der Klebstoff ausgewählt aus einem Acrylat und/oder einem Polyurethan und/oder einem Epoxidharz und/oder einem Cyanacrylat.

Gemäß einem weiteren Aspekt der vorliegenden Erfindung, der mit den vorherigen Aspekten kombinierbar ist, umfasst eine Plasmabehandlungsvorrichtung für Glasspritzenkörper hypodermischer Glasspritzen eine Atmosphärendruckplasmaquelle zum Bereitstellen von Plasma zum Behandeln einer Innenfläche eines Spritzenkonus des Glasspritzenkörpers. Vorzugsweise handelt es sich bei dem Glasspritzenkörper um einen vorab mittels einer Gleitmittelschicht, wie einer Silikonschicht, beschichteten Glasspritzenkörper. Beispielsweise kann der Glasspritzenkörper auch einbrennsilikonisiert worden sein. Durch die Plasmabehandlung lässt sich die Wirkung der Beschichtung zumindest teilweise deaktivieren bzw. rückgängig machen, ohne die Beschichtung als solche vollständig zu entfernen. Durch die Plasmabehandlung wird die jeweilige Oberfläche, d. h. eine Glaskörperinnenfläche derart bearbeitet, dass die Haftfestigkeit bzw. die Benetzbarkeit mit einem Schmiermittel, einem Klebstoff, einem Lot oder einem Lack ansteigt bzw. überhaupt erst ermöglicht wird.

Ferner umfasst die Plasmabehandlungsvorrichtung eine Unterdruckquelle zum Bereitstellen eines Unterdrucks kleiner als Atmosphärendruck. Beispielsweise kann ein Vakuum generiert werden. Erfindungsgemäß sind die Atmosphärendruckplasmaquelle und die Unterdruckquelle relativ zu dem Spritzenkonus einander gegenüberliegend angeordnet bzw. anordenbar. Insbesondere ist die Atmosphärendruckplasmaquelle an derjenigen Seite des Spritzenkonus angeordnet, an welcher die innerhalb der Spritze untergebrachte Flüssigkeit die Spritze verlässt, und die Unterdruckquelle an derjenigen Seite der Spritze angeordnet, über welche die Spritze mit der Flüssigkeit befüllt wird. Durch das Bereitstellen der Unterdruckquelle wird das Eindringen des Plasma als solches bzw. eine Steigerung der Eindringtiefe in den Spritzenkonus ermöglicht. Dadurch ist eine definierte Behandlung des Spritzenkonusinnenbereichs mit reinem Atmosphärenplasma ermöglicht. Folglich ist eine Steigerung der Adhäsion von Klebstoff und Glas zu erzielen, wodurch eine erhöhte Haltekraft zwischen anzubringender Injektionsnadel bzw. Kanüle und Glasspritze erreicht wird. Zusätzlich bewirkt die Bereitstellung von Unterdruck auch eine Abdichtungswirkung, da vermieden wird, dass das Plasma während der Behandlung des Spritzenkörpers in diesen gelangt und dort zu einer unerwünschten Behandlung von weiteren Bereichen des Spritzenkörperinnenbereichs führt.

In einer beispielhaften Ausführung der vorliegenden Erfindung umfasst die Unterdruckquelle der Plasmabehandlungsvorrichtung einen Saugdorn, dessen Außendurchmesser kleiner oder gleich einem Kolbenraum-Innendurchmesser des Glasspritzenkörpers, insbesondere des Kolbenraums, ist. Der Saugdorn dient beispielsweise der gezielten Anwendung von Unterdruck und kann vorzugsweise derart ausgebildet sein, dass eine Stärke bzw. ein Ausmaß des Unterdrucks über diesen geregelt bzw. eingestellt werden kann.

In einer weiteren beispielhaften Ausführung der erfindungsgemäßen Plasmabehandlungsvorrichtung weist der Saugdorn eine Einführlänge auf, entlang welcher der Außendurchmesser kleiner als der Kolbenraum-Innendurchmesser des Glasspritzenkörpers ist. Die Einführlänge wird beispielsweise in Axialrichtung gemessen. Die Einführlänge kann dabei wenigstens so lang sein, wie die Zylinderhöhe des Kolbenraums. Die Plasmabehandlungsvorrichtung kann außerdem dazu eingerichtet sein, den Saugdorn in den Kolbenraum des Gasspitzenkörpers einzuführen, um dort einen Unterdruck anzulegen. Vorzugsweise ist der Saugdorn in den Kolbenraum bis in einen Berührkontakt mit einer Bodenfläche des Kolbenraums einführbar. Dadurch kann zusätzlich eine Abdichtwirkung zwischen Glaskörperinnenfläche, insbesondere der Bodenfläche, und dem Saugdorn erzielt werden, insbesondere um zu vermeiden, dass Plasma während der Behandlung in den Spritzenkörper gelangt.

Gemäß einer Weiterbildung der vorliegenden Erfindung weist der Saugdorn ein Dichtmittel, wie einen Dichtring und/oder einen Dichtschlauch, auf. Das Dichtmittel ist beispielsweise am Stirnende, welches in Axialrichtung gesehen in Richtung des Spritzenkonus gewandt ist, angeordnet. Insbesondere ist das Dichtmittel dazu vorgesehen, in einen Berührkontakt mit einem Innendurchmesser, also einer Kolbenraum-Innenfläche, und/oder einer Bodenfläche des Kolbenraums gebracht zu werden.

Im Folgenden werden weitere Eigenschaften, Merkmale und Vorteile der Erfindung mittels Beschreibung bevorzugter Ausführungen der Erfindung anhand der beiliegenden beispielhaften Zeichnungen deutlich, in denen zeigen:
- Fig. 1: eine Querschnittsansicht einer erfindungsgemäßen hypodermischen Fertigglasspritze;
- Fig. 2: eine Teil-Querschnittsansicht einer erfindungsgemäßen hypodermischen Fertigglasspritze mit Plasmaquelle und Unterdruckquelle;
- Fig. 3: einen Ausschnitt einer erfindungsgemäßen Plasmabehandlungsvorrichtung;
- Fig. 4: eine beispielhafte Ausführung einer erfindungsgemäßen Plasmabehandlungsvorrichtung;
- Fig. 5: eine beispielhafte Ausführung einer Plasmaquelle;
- Fig. 6: ein Diagramm einer beispielhaften Schichtdickenabnahme über die Behandlungszeit;
- Fig. 7: eine weitere Ausführung einer erfindungsgemäßen hypodermischen Fertigglasspritze; und
- Fig. 8: eine weitere Ausführung einer erfindungsgemäßen hypodermischen Fertigglasspritze.

In den folgenden Darstellungen bevorzugter Ausführungen werden für dieselben oder ähnliche Komponenten dieselben oder ähnliche Bezugszeichen verwendet. Eine erfindungsgemäße hypodermische Fertigglasspritze ist im Allgemeinen mit der Bezugsziffer 7 versehen. Eine erfindungsgemäße Plasmabehandlungsvorrichtung für Glasspritzenkörper hypodermischer Fertigungsspritzen ist im Allgemeinen mit der Bezugsziffer 100 versehen.

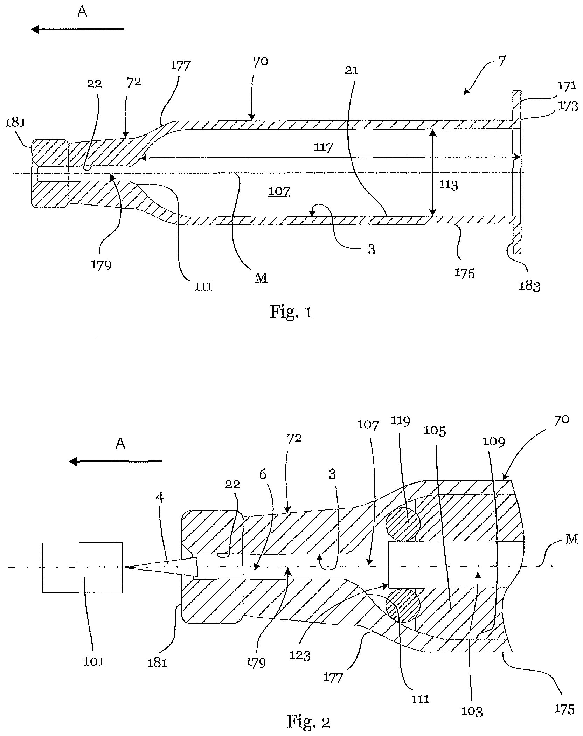

In Fig. 1 ist eine erfindungsgemäße hypodermische Fertigglasspritze 7 schematisch in einer Schnittansicht dargestellt, wobei ein Glasspritzenkörper 70 der Fertigglasspritze 7 schraffiert dargestellt ist. Die Glasspritze 7 bzw. der Glasspritzenkörper 70 umfasst im Wesentlichen einen Flanschabschnitt 171 an einem in Axialrichtung A betrachteten Ende 173, einen länglichen, im Wesentlichen querschnittskonstanten sowie wandstärkekonstanten Kolbenabschnitt 175, der in Axialrichtung A betrachtet an den Flanschabschnitt 171 anschließt, und einen sich im Querschnitt verjüngenden, mit einer dickeren Wandstärke ausgebildeten Spritzenkonus 72, in den der Kolbenabschnitt 175 in Axialrichtung A mündet. Innenseitig begrenzt bzw. definiert der Kolbenabschnitt 175 einen Kolbenraum 107. Angrenzend an den Spritzenkonus 72 schließt ein Trichterabschnitt 177 des Kolbenabschnitts 175 an, in dessen Innerem sich das Kolbenvolumen 107 trichterartig verjüngt. Durch den Spritzenkonus 72 erstreckt sich im Wesentlichen in Axialrichtung A ein Durchgang 179 von einem, dem Flanschende 173 gegenüberliegenden Spritzenkonus-Ende 181 bis hin zu dem Kolbenraum 107. Der Durchgang bzw. Endkanal 179 besitzt einen im Wesentlichen konstanten Querschnitt während seines Verlaufs. An dem Spritzenkonus-Ende 181 kann der Durchgang 179 angefast sein, beispielsweise mit einer Fase mit einem Winkel von 45° bezüglich der Axialrichtung A, um das spätere Einsetzen einer Kanüle bzw. einer Injektionsnadel und/oder einer Atmosphärendruckplasmaquelle 101, die weiter unten im Detail beschrieben wird, zu erleichtern. In Fig. 1 ist eine Mittel- bzw. Symmetrielinie M durch eine Strichpunktlinie angedeutet, die im Wesentlichen parallel zur Axialrichtung A orientiert ist. Es ist zu erkennen, dass die Fertigglasspritze 7 bezüglich der Mittellinie M symmetrisch aufgebaut ist. Innenseitig, d.h. an einer Innenfläche 21 der Fertigglasspritze 7, ist eine Beschichtung 3, die vorzugsweise als Einbrennsilikonisierung ausgestaltet ist, aufgebracht. Dadurch wird insbesondere gewährleistet, dass ein einzuschiebender Kolben 71, der näher weiter unten beschrieben wird, bezüglich der Innenfläche 21 der Glasspritze 7 möglichst reibungsarm und somit gut gleitend entlanggeschoben werden kann. Eine Teilfläche 22 der Innenfläche 21, die, wie insbesondere in Fig. 1 zu sehen ist, wenigstens einen Teil der Spritzenkonus-Innenfläche bzw. der Innenfläche des Durchgangs 179 bildet, ist vordergründlich als spätere Klebefläche für die einzuklebende Injektionsnadel 73 bzw. Kanüle 76 vorgesehen. Es sei klar, dass auch die Teilfläche 22 mit der Beschichtung 3 durch die Atmosphärendruckplasmaquelle 101 behandelt ist. Ferner ist es vorzugsweise die Teilfläche 22, welche unter Anwendung des erfindungsgemäßen Beschichtungsverfahrens mit der Unterdruckquelle 103 behandelt wird, um die Teilfläche 22 derart zu bearbeiten, dass eine erhöhte Adhäsion des zum Kleben verwendeten Klebstoffs erwirkt. Das Behandeln des Spritzeninneren mit der Atmosphärendruckplasmaquelle 101 und der Unterdruckquelle 103 wird in Bezug auf Fig. 2 bis 4 näher beschrieben.

Der Flanschabschnitt 171 besitzt wenigstens einen zumindest teilweise in Umfangsrichtung sich erstreckenden, und sich senkrecht zur Axialrichtung von der Mittelachse M weg erstreckenden Flanschsteg 183, welcher zum Betätigen mit den Fingern durch einen Benutzer vorgesehen ist.

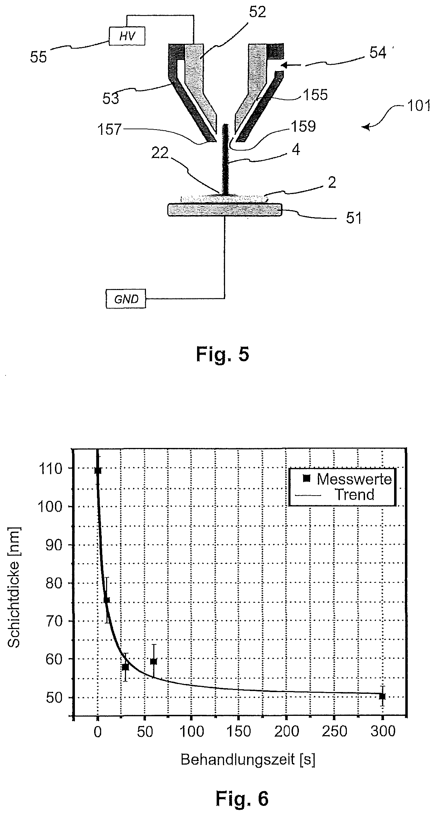

In Fig. 2 ist ein Ausschnitt einer erfindungsgemäßen Fertigglasspritze 7 schematisch dargestellt, wobei an dem spritzenkonusseitigen Ende 181 eine Atmosphärendruckplasmaquelle 101 angeordnet ist und kolbenabschnittseitig eine Unterdruckquelle 103 im Inneren des Kolbenraums 107 sich befindet. Hinsichtlich der Details zu einer bevorzugten Ausführung der Atmosphärendruckplasmaquelle 101 wird auf Fig. 5 verwiesen. In Fig. 2 ist diese lediglich als Block dargestellt, wobei schematisch angedeutet ist, dass ein Plasmastrahl 4 über das spritzenkonusseitige Ende 181 in den Endkanal 179 in dem Spritzenkonus 72 eingebracht wird, um die Innenfläche 21 sowie die Teilfläche 22 mit dem Plasmastrahl 4 zu behandeln. Erfindungsgemäß wird das Plasmabehandeln der Innenflächen der Fertigglasspritze 7 mit dem Anlegen einer Unterdruckquelle 103 vorzugsweise im Spritzeninneren kombiniert. Von der Unterdruckquelle 103 ist in Fig. 2 lediglich ein Saugdorn 105, eingeführt in den Kolbenraum 107 des Glasspritzenkörpers 70, dargestellt. In der dargestellten Position des Saugdorns 105 bezüglich des Glasspritzenkörpers 70 befindet sich der Saugdorn 105 in einem Berührkontakt mit einer Innenseite 109 des Glasspritzenkörpers 70, wobei es sich hierbei um einen gleitenden Berührkontakt handelt, und einer ansatzweise trichterförmigen Bodenfläche 111 des Kolbeninnraums 107, welche ein weiteres Einführen des Saugdorns 105 in den Kolbenraum 107 verhindert. Gemäß der Ausführungsform in Fig. 2 ist ein Dichtring 119 an einem in Axialrichtung A in Richtung der Bodenfläche 111 gewandten Stirnende 123 des Saugdorns 105 angeordnet, insbesondere um einen abdichtenden Berührkontakt mit der Bodenfläche 111 bereitzustellen. Alternativ oder zusätzlich zu dem Dichtring 119 kann auch ein Dichtungsschlauch (nicht dargestellt) zur Bereitstellung einer zusätzlichen Abdichtwirkung an dem Saugdorn angebracht sein. Es ist zu erkennen, dass durch die Unterdruckquelle 103 bzw. durch den Saugdorn 105 der Unterdruckquelle 103 Unterdruck kleiner als Atmosphärendruck im Bereich des Spritzenkonus 72, d.h. im Wesentlichen in dem Durchgang 179 erzeugt wird, um die Teilfläche 22 der Innenfläche 21 der Fertigglasspritze 7 sowohl mit Atmosphärendruckplasma als auch mit Unterdruck zu beaufschlagen. Dadurch stellt sich die erfindungsgemäße vorteilhafte Eigenschaftsveränderung an der Teilfläche 22 ein, um das nachträgliche Einkleben der Injektionsnadel 73 bzw. der Kanüle 76 zu ermöglichen bzw. zu verstärken und die Haltekraft bezüglich der Glaskörperinnenseite 22 in Axialrichtung A zu erhöhen.

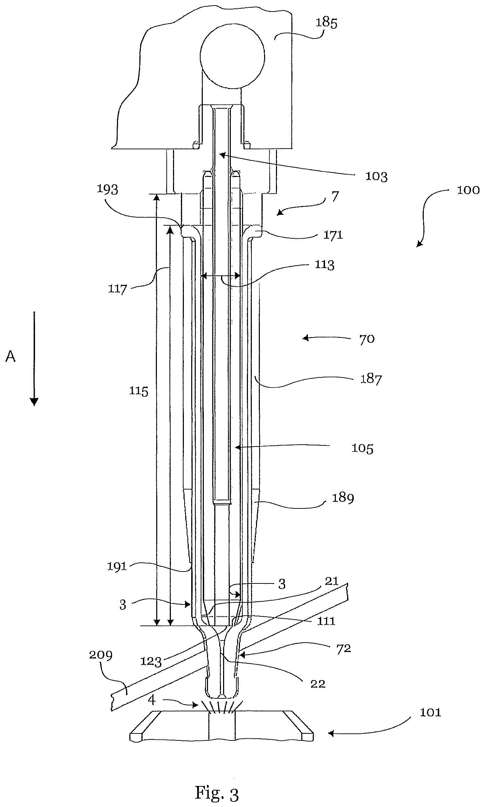

In Fig. 3 ist ein Ausschnitt einer Plasmabehandlungsvorrichtung 100 für Glasspritzenkörper 70 hypodermischer Fertigglasspritzen 7 in einer Seitenansicht dargestellt. Die Plasmabehandlungsvorrichtung 100 umfasst ein Traggestell 185, an dem wenigstens ein Greifer 187 zum Greifen bzw. Halten eines Glasspritzenkörpers 70 einer hypodermischen Fertigglasspritze 7 angeordnet ist. Der Greifer 187 erstreckt sich in Axialrichtung A betrachtet etwa um Dreiviertel der vollständigen Längserstreckung des Glasspritzenkörpers 70. Allerdings kann sich der Greifer 187 auch beispielsweise um 15 %, 30 %, 45 %, 60 % oder auch zwischen 80 % oder 100 % der vollständigen Längserstreckung des Glasspritzenkörpers 70 in Axialrichtung A erstrecken. Der Greifer besitzt eine im Wesentlichen konstante Querschnittsform, wobei an dem dem Traggestell 185 abgewandten Ende eine langgezogene Fase 189 vollumfänglich des Greifers 187 ausgebildet ist. Der Greifer 187 besitzt im Wesentlichen eine Zylinderform, wobei im Wesentlichen entlang seiner gesamten Längserstreckung, d.h. in Axialrichtung A in Fig. 3, ein Schlitz 191 zum seitlichen Einschieben eines Glasspritzenkörpers 70 vorgesehen ist. Im montierten Zustand des Glasspritzenkörpers 70 in dem Greifer 187 umgreift dieser den Glasspritzenkörper 70 dabei schellenartig, wobei ein Innendurchmesser des Greifers 187 etwa dem Außendurchmesser des Glasspritzenkörpers 70 entspricht, wobei klar ist, dass der Innendurchmesser des Greifers 187 nicht kleiner als der Außendurchmesser des Glasspritzenkörpers 70 sein sollte. Es ist allerdings möglich, eine Presspassung zwischen Glasspritzenkörper 70 und Greifer 187 vorzusehen. Des Weiteren besitzt der Greifer 187 nahe des Traggestells 185 zwei senkrecht zur Axialrichtung A verlaufende Nuten 193, in die der Flanschabschnitt 177 des Glasspritzenkörpers 70 einzuschieben ist. Wie insbesondere in Fig. 3 zu sehen ist, ist die Dimensionierung der Schlitze 193 derart gewählt, dass der Flanschabschnitt 171 passend aufgenommen ist. Beidseitig in Bezug auf den Glasspritzenkörper 70 ist jeweils ein Schlitz 193 in den Greifer 187 eingebracht.

Unterhalb des Glasspritzenkörpers 70 ist ein Atmosphärenplasmadruckquelle 101 der Plasmabehandlungsvorrichtung 100 zum Bereitstellen von Plasma zum Behandeln einer Innenfläche 21 des Glasspritzenkörpers 70 angeordnet, wobei mittels der Bezugsziffer 4 ein Plasmastrahl angedeutet ist, der in das Spritzeninnere gerichtet ist. Die Atmosphärendruckplasmaquelle 101 wird in Bezug auf Fig. 5 (weiter unten) näher erläutert.

An dem Traggestell 185 selbst kann eine Unterdruckquelle 103 zum Bereitstellen eines Unterdrucks kleiner als Atmosphärendruck angeordnet sein. Die Atmosphärendruckquelle 101 und die Unterdruckquelle 103 sind relativ zu dem Spritzenkonus 72 des Glasspritzenkörpers 70 einander gegenüberliegend anordenbar, so dass in dem Spritzenkonus 72 der Unterdruck zur erfindungsgemäßen Behandlung wenigstens einer Teilfläche 22 der Innenfläche 21 des Glasspritzenkörpers 70 durchgeführt werden kann. Die Unterdruckquelle 103 kann beispielsweise auch an anderer Position, als an dem Traggestell 185 angebracht sein, wobei wenigstens ein Applikator, wie ein Saugdorn 105 der Unterdruckquelle 103 erfindungsgemäß relativ zu dem Spritzenkonus 72 der Atmosphärendruckplasmaquelle 101 gegenüberliegend anordenbar sein muss. Wie in Fig. 3 dargestellt, besitzt die Unterdruckquelle 103 einen Saugdorn 105 mit einem Außendurchmesser kleiner oder gleich einem Kolbenrauminnendurchmesser 113 des Glasspritzenkörpers 70, so dass dieser in die erfindungsgemäße Behandlungsposition bringbar ist. Des Weiteren ist in Fig. 3 zu erkennen, dass der Saugdorn 105 eine Einführlänge 115 besitzt, entlang welcher der Außendurchmesser kleiner als der Kolbenrauminnendurchmesser 113 des Glasspritzenkörpers 70 ist. In Fig. 3 ist der Saugdorn 105 bis zu einem Berührkontakt mit einer Bodenfläche 111 des Kolbenraums 107 eingeführt. Mit anderen Worten ist ein Stirnende 123 des Saugdorns 105 derart weit in den Kolbenraum eingeschoben, dass das Stirnende 123 auf der Bodenfläche 111 des Kolbenraums 107 aufliegt.

Das Vorhandensein eines Dichtmittels, wie eines Dichtrings 119 und/oder eines Dichtschlauchs (nicht dargestellt), welches beispielsweise an dem Stirnende 123 des Saugdorns 105 anzuordnen ist, kann vorteilhaft sein, um in einen abdichtenden Berührkontakt mit der Innenseite 109 und/oder der Bodenfläche 111 des Kolbenraums 107 gebracht zu werden.

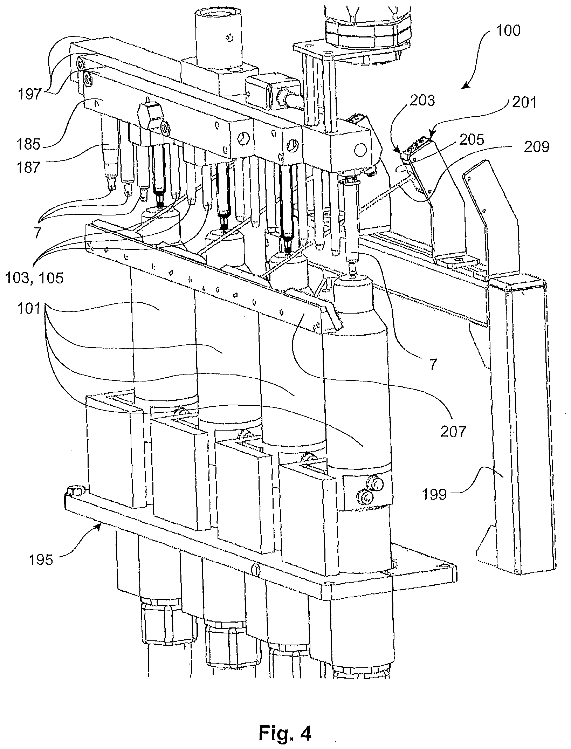

In Fig. 4 ist eine beispielhafte Ausführung einer erfindungsgemäßen Plasmabehandlungsvorrichtung 100 dargestellt. In der folgenden Beschreibung werden lediglich die Komponenten beschrieben, die noch nicht bereits in Bezug auf die vorherigen Fig. 1 bis 3 erläutert wurden. Die beispielhafte Plasmabehandlungsvorrichtung 100 besitzt eine Plasmaquellenanordnung 195 von vier Atmosphärendruckplasmaquellen 101 zum gleichzeitigen oder aufeinanderfolgenden Behandeln von Glasspritzenkörpern 70 hypodermischer Fertigglasspritzen 7. An dem Traggestell 185, welches beispielsweise aus drei aneinander montierten Längsstreben 197 bestehen kann, können beispielsweise mindestens fünf und beispielsweise höchstens 30 Glasspritzenkörper 70 hypodermischer Glasspritzen 7 haltend angebracht werden, um diese mittels der Plasmabehandlungsvorrichtung 100 zu behandeln. Außerdem ist die Plasmabehandlungsvorrichtung 100 derart ausgebildet, dass entsprechend der Anzahl an aufnehmbaren Glasspritzenkörpern 70 Unterdruckquellen 103 vorgesehen sind, bzw. Saugdorne 105 einer gemeinsamen oder separater Unterdruckquellen 103.

An einem Ständer 199, der beispielsweise türrahmen- oder torbogenartig ausgebildet sein kann, ist wenigstens eine, bzw. drei gleich ausgebildete (Fig. 4), Sensoreinrichtung 201 angebracht, welche dazu dient, zu erfassen, ob sich ein Glasspritzenkörper 70 in einem Greifer 187 des Traggestells 185 befindet. Anhand dieser Erkenntnis kann dann diejenige Atmosphärendruckplasmaquelle 101 und diejenige Unterdruckquelle 103 angesteuert werden, mittels denen der entsprechende Glasspritzenkörper 70 behandelt werden kann. Beispielsweise ist die Sensoreinrichtung 201 als Lasereinrichtung 203 ausgebildet und besitzt einen Laserstrahlerzeuger 205, der an dem Ständer 199 angebracht ist, sowie eine Reflexionseinrichtung 207, beispielsweise eine Spiegeleinrichtung, zum Reflektieren eines von der Lasereinrichtung 205 erzeugten Laserstrahls 209. Es sei klar, dass weitere Sensoreinrichtungen 201 denkbar sind, die dazu in der Lage sind, zu erfassen, ob sich ein Glasspritzenkörper 70 in einer jeweiligen Halterung 187 des Traggestells 185 befindet.

Anhand von Fig. 5 wird eine Plasmaquelle näher erläutert, welche zur Durchführung des vorstehend beschriebenen Beschichtungsverfahrens geeignet ist und beispielhaft als Atmosphärendruckplasmaquelle 101 ausgebildet ist. Die Atmosphärendruckplasmaquelle 101 umfasst eine Hochspannungselektrode 52, welche von einem Isolator 53 umgeben ist. Die Hochspannungselektrode 52 ist als Hohlkörper ausgebildet und kann beispielsweise zylindrisch oder konisch sein. Der Isolierkörper 53 ist von der Hochspannungselektrode 52 beabstandet angeordnet. Auf diese Weise kann ein Arbeitsgas in den Zwischenraum 155 zwischen der Hochspannungselektrode 52 und dem Isolierkörper 53 eingeführt werden, welches über eine Gaszufuhr 54 zugeführt werden kann. Das Arbeitsgas verlässt die Hochspannungselektrode 52 über deren substratseitige Mündung 157, die trichterartig ausgebildet ist und einen sich im Längsverlauf verringernden Querschnitt besitzt und eine Austrittsöffnung 159 definiert, über welche das Arbeitsgas die Mündung 157 verlassen kann.

Der Mündung 157 gegenüberliegend ist eine Gegenelektrode 51 angeordnet, welche optional mit einer dielektrischen Beschichtung versehen sein kann. Hierdurch wird sichergestellt, dass zwischen der Hochspannungselektrode 52 und der Gegenelektrode 51 in jedem Fall eine dielektrisch behinderte Entladung gezündet wird. Sofern das Substrat selbst ein Dielektrikum bzw. einen Isolator enthält oder daraus besteht, kann die dielektrische Beschichtung der Gegenelektrode 51 auch entfallen.

Bei Betrieb der Vorrichtung wird über die Gaszufuhr 54 ein Arbeitsgas, beispielsweise Argon, zugeführt. An die Hochspannungselektrode 52 wird eine hochfrequente Wechselspannung angelegt, welche mit einer Hochspannungsquelle 55 erzeugt wird. In einigen Ausführungsformen der Erfindung kann die Amplitude der angelegten Hochspannung zwischen etwa 2 kV bis etwa 10 kV oder zwischen etwa 5 kV und etwa 8 kV betragen. Die Hochspannung kann als sinusförmige Wechselspannung angelegt werden oder aber in Form einzelner Hochspannungspulse. Die Pulsfolgefrequenz bzw. die Wechselspannungsfrequenz kann zwischen etwa 10 Hz und etwa 30 kHz betragen. Die im Plasma 4 umgesetzte Leistung kann mittels eines Messkondensators bestimmt werden, welcher die transferierten Ladungsträger eines Entladungszyklus integriert. Die so bestimmte Leistung kann zwischen etwa 0,5 Watt und etwa 5 Watt oder zwischen etwa 1 Watt und etwa 3 Watt betragen.

Der auf diese Weise erzeugte Plasmastrahl weist einen Durchmesser von etwa 0,15 mm bis etwa 0,5 mm auf. Beim Auftreffen auf ein beispielhaftes Bauteil 2 weitet sich der Fußpunkt auf, sodass die Teilfläche 22 größer sein kann als der Durchmesser des Plasmastrahles 4. Sofern die Teilfläche 22 größer ist als der sich aus der Geometrie der Plasmaquelle ergebende Strahlfleck, kann durch Verschieben des Bauteils 2 bzw. der Gegenelektrode 51 mit dem darauf angeordneten Bauteil 2 eine größere Teilfläche 22 durch sequenzielles Behandeln mit dem Plasma 4 bearbeitet werden. Der Abstand des Strahlaustritts von der zu behandelnden Oberfläche kann zwischen etwa 3 mm und etwa 8 mm betragen.

Anhand von Fig. 6 wird die elipsometrisch bestimmte Schichtdickenabnahme der Teilfläche 22 durch die Plasmabehandlung erläutert. Dabei ist die Behandlungszeit mit dem anhand von Fig. 5 erläuterten Plasmastrahl 4 auf der Abszisse aufgetragen sowie die elipsometrisch bestimmte Schichtdicke auf der Ordinate.

Wie Fig. 6 zeigt, beträgt die Schichtdicke vor Einwirkung des Plasmas 110 nm. Nach einer Einwirkdauer von etwa 10 Sekunden verringert sich die Schichtdicke bereits auf etwa 75 nm. Nach 30 s beträgt die Schichtdicke etwa 57 nm. Bei sehr langer Behandlungsdauer von 300 Sekunden nimmt die Schichtdicke auf etwa 50 nm ab. Die Schichtdicke zeigt mit der Behandlungszeit einen asymptotischen Verlauf. Die in Fig. 6 gezeigten Messwerte lassen vermuten, dass die Schichtdicke auch bei noch längerer Behandlungszeit nicht unter 50 nm fällt.

Damit zeigt Fig. 6, dass mit der erfindungsgemäßen Plasmabehandlung der Beschichtung keine vollständige Entfernung der Beschichtung erreicht wird. Gleichwohl ändert sich die chemische Zusammensetzung und/oder die Bindungsverhältnisse der Konstituenten innerhalb der Beschichtung, wie nachfolgend noch näher erläutert werden wird. Damit einher geht eine Änderung des Benetzungsverhaltens. Die mit dem Plasma behandelte zweite Teilfläche 22 ist nicht mehr wie ursprünglich hydrophob, sondern hydrophil, sodass die Teilfläche 22 nach Einwirkung des Plasmas 4 dazu geeignet ist, mittels einer Klebeverbindung gefügt oder nochmals mit einem anderen Beschichtungsmaterial beschichtet zu werden.

Die in Fig. 6 gezeigten Messwerte wurden anhand einer Beschichtung 3 ermittelt, welche durch Einbrennsilikonisierung erhalten wurde. Hierzu wird eine Emulsion aus Silikonöl und Wasser aufgetragen und nachfolgend durch eine Wärmebehandlung auf die Oberfläche des Bauteils 2 eingebrannt. Die nachfolgende Tabelle zeigt das Elementinventar der Beschichtung 3 vor der Einwirkung des Plasmas, nach 1 Sekunde, 10 Sekunden, 30 Sekunden, 60 Sekunden und nach 300 Sekunden. Alle Messwerte wurden mittels Photoelektronenspektroskopie erhalten. Dabei wird monochromatische Röntgenstrahlung auf die Oberfläche der Beschichtung 3 eingestrahlt und die kinetische Energie der Photoelektronen bestimmt. Aus der kinetischen Energie kann das jeweilige Element bestimmt werden, die Intensität der Photoelektronen gibt die relativen Anteile in der Beschichtung 3 an.

**Tabelle 1: Elementinventar der Beschichtung**

| Element | 0 s [Atom-%] | 1 s [Atom-%] | 10 s [Atom-%] | 30 s [Atom-%] | 60 s [Atom-%] | 300 s [Atom-%] |
|---|---|---|---|---|---|---|
| Sauerstoff (O) | 39,1 | 56,2 | 60,4 | 67 | 66,96 | 66,96 |
| Kohlenstoff (C) | 35,8 | 18,93 | 13,9 | 5,6 | 4,14 | 3,63 |
| Silizium (Si) | 25,1 | 24,85 | 25,7 | 27,4 | 28,68 | 29,28 |
| Rest | -- | -- | -- | -- | 0,27 | 0,13 |

Die Messungen wurden nach Einwirkung eines Atmosphärendruck-Plasmastrahls 4 mit atmosphärischer Luft als Arbeitsgas erhalten, welcher beispielsweise mit der Vorrichtung nach Fig. 5 erhältlich ist. Wie die vorstehend dargestellten Messwerte zeigen, nimmt der Kohlenstoffgehalt der Beschichtung mit zunehmender Einwirkdauer des Plasmas rasch ab. Dies ist darauf zurückzuführen, dass die im Silikon enthaltenen Methylgruppen abgespalten und durch den Gasstrom des Arbeitsgases des Plasmas 4 abtransportiert werden.

Um die Nadelhaltekraft zwischen Injektionsnadel 73 und Fertigglasspritze 7 zu erhöhen, insbesondere um Festigkeitswerte im Rahmen der DIN ISO 7864 zu gewährleisten, wird der erfindungsgemäße Verfahrensschritt des Anordnens einer Unterdruckquelle 103 gegenüberliegend der Atmosphärendruckplasmaquelle 101, relativ zu dem Spritzenkonus 72 betrachtet, angewendet. Die Beschichtung einer Teilfläche 22 ist nach dem Behandeln mit Plasma 4 beim gleichzeitigen Anlegen von Unterdruck durch die Unterdruckquelle 103 derart bearbeitet, dass eine nachträgliche Adhäsion eines aufzubringenden Klebstoffs an der Teilfläche 22 deutlich verstärkt ist, um damit die Haltekraft zwischen Kanüle 76 bzw. Injektionsnadel 73 und Fertigglasspritze 7 zu erhöhen.

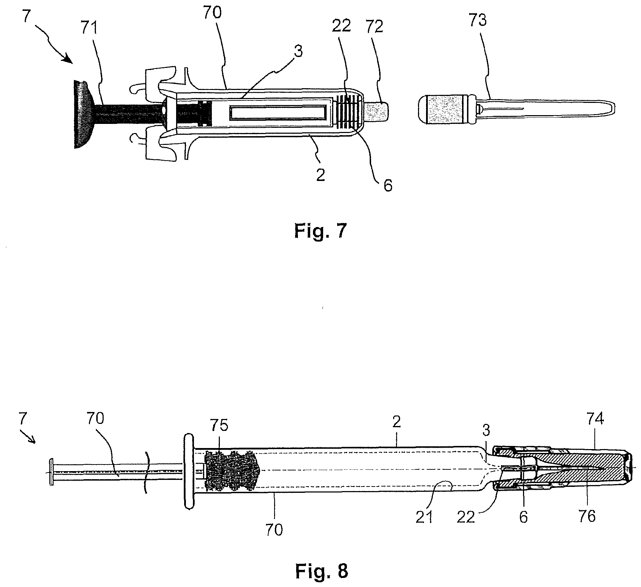

Fig. 7 zeigt eine Fertigspritze, die beispielhaft als Fertigglasspritze 7 ausgebildet ist, als weiteres Ausführungsbeispiel der Erfindung. Die Fertigglasspritze 7 weist einen in etwa zylindrischen Spritzenkörper 70 auf. Fertigglasspritzen 7 der dargestellten Art dienen als Verpackung des darin enthaltenen Medikamentes im Herstellwerk, sodass dieses unmittelbar gebrauchsfertig an den Arzt bzw. Patienten übergeben werden kann.

Zur Herstellung (nicht dargestellt) der in Fig. 7 dargestellten Fertigglasspritze 7 wird zunächst der Spritzenkörper 70 aus einem Glasrohr gefertigt. Hierzu wird das Glasrohr zugeschnitten, erwärmt und entsprechend der gewünschten Form umgeformt.

Im nächsten Verfahrensschritt wird zumindest die Innenseite mit einer Emulsion aus einem Lösungsmittel und schichtbildenden Substanzen behandelt, wie besprüht, und nachfolgend in einem Ofen bzw. Wärmeschrank behandelt. Dies führt dazu, dass ein Großteil des in der Emulsion vorhandenen Lösungsmittels verdampft. Gleichzeitig wird das als schichtbildende Substanz enthaltene Silikon kovalent an das Glas gebunden, sodass sich eine Beschichtung 3 auf der Innenseite ausbildet, welche Polysiloxan enthält oder daraus besteht. Durch die Wärmebehandlung wird vermieden, dass das Silikon beim späteren Befüllen, bei der Lagerung und beim Gebrauch der Fertigglasspritze 7 unerwünschterweise in das Medikament übergeht. Gleichzeitig erlaubt die Silikonisierung ein leichtes Gleiten des Kolbens 7, sodass die Handhabung der Fertigglasspritze 7 erleichtert ist.

In der dargestellten Ausführungsform wird an dem dem Kolben 71 gegenüberliegenden Ende des Spritzenkörpers 70 ein Konus 72 eingeklebt, welcher zur Aufnahme der Injektionsnadel 73 vorgesehen ist. In anderen Ausführungsformen der Erfindung kann die Injektionsnadel 73 auch unmittelbar in den Spritzenkörper 70 eingeklebt werden, sodass der Konus 72 auch entfallen kann, oder alternativ der Konus 72 aus einem Stück mit dem Glasspritzenkörper 70 hergestellt ist.

Da die Beschichtung 3 auch die zur Aufnahme des Konus 72 vorgesehene Teilfläche 22 bedeckt, ist die Haftfestigkeit einer Klebeverbindung 6 reduziert. Dies kann so weit gehen, dass der Konus 72 bereits beim Transport oder der Lagerung aus dem Spritzenkörper 70 herausfällt und der Inhalt der Fertigglasspritze 7 ausläuft.

Erfindungsgemäß wird daher vorgeschlagen, die Teilfläche 22 in der vorstehend beschriebenen Weise mit einem Atmosphärendruckplasma zu behandeln und hierdurch die Beschichtung 3 zwar nicht vollständig zu entfernen, jedoch soweit zu inaktivieren, dass die Klebeverbindung 6 zuverlässig ausgefüllt werden kann. Dies erfolgt erfindungsgemäß durch das Anlegen einer Unterdruckquelle 103, die relativ zu dem Spritzenkonus 72 in Axialrichtung A gegenüberliegend zu der Atmosphärendruckplasmaquelle 101 angeordnet ist und einen Unterdruck kleiner als Atmosphärendruck bereitstellt. Durch die Änderung des Elementinventars und/oder der Bindungsverhältnisse der Konstituenten kann die hydrophobe Beschichtung 3 in der Teilfläche 22 hydrophil werden, um die Haftfestigkeit der Verklebung 6 deutlich zu verbessern, bzw. Nadelhaltekräfte in Axialrichtung A bereitzustellen, die den Anforderungen der DIN ISO 7864 genügen.

Fig. 8 zeigt eine Fertigspritze, die beispielsweise ebenfalls als Fertigglasspritze 7 ausgebildet sein kann und deren prinzipielle Funktion bereits anhand den vorherigen Ausführungsformen erläutert wurde. Gleiche Bestandteile sind mit gleichen Bezugszeichen versehen, so dass sich die nachstehende Beschreibung auf die wesentlichen Unterschiede beschränkt.

Zur Herstellung der in Fig. 8 dargestellten Fertigglasspritze 7 (Staked In Needle Spritze) wird zunächst der Spritzenkörper 70 aus einem Glasrohr gefertigt. Hierzu wird das Glasrohr zugeschnitten, erwärmt und entsprechend der gewünschten Form umgeformt. Der Spritzenkörper 70 kann jedoch auch aus Kunststoff bestehen (nicht dargestellt). Kunststoffspritzen werden durch bekannte Herstellverfahren, z.B. Spritzguss, gefertigt.

Im nächsten Verfahrensschritt wird zumindest die Innenseite des Spritzenkörpers 70 mit einer Emulsion aus einem Lösungsmittel und schichtbildenden Substanzen besprüht und nachfolgend in einem Ofen, z.B. einem Tunnelofen oder Wärmeschrank behandelt. Dies führt dazu, dass ein Großteil des in der Emulsion vorhandenen Lösungsmittels verdampft. Gleichzeitig wird das als schichtbildende Substanz enthaltene Silikon größtenteils kovalent an das Glas gebunden, sodass sich eine Beschichtung 3 auf der Innenseite, das heißt auf der Innenfläche 21 inklusive der Teilfläche 22, ausbildet, welche Polysiloxan und/oder Polydimethylsiloxan enthält oder daraus besteht. Durch die Wärmebehandlung wird vermieden, dass das Silikon beim späteren Befüllen, bei der Lagerung und beim Gebrauch der Fertigglasspritze 7 unerwünscht in das Medikament übergeht. Gleichzeitig erlaubt die Silikonisierung ein leichtes Gleiten des Kolbenstopfens 75, sodass die Handhabung der Fertigglasspritze 7 bzw. eine Applikation des Medikamentes erleichtert oder überhaupt erst möglich wird.

In der dargestellten Ausführungsform wird an dem, dem Kolbenstopfen 75 gegenüberliegenden, Ende des Spritzenkörpers 70, eine Kanüle 76 eingeklebt. Da die Beschichtung 3 auch die zur Aufnahme der Kanüle 76 vorgesehene Teilfläche 22 bedeckt, ist die Haftfestigkeit einer Klebeverbindung 6 reduziert. Dies kann so weit gehen, dass die Kanüle 76 bereits beim Transport oder der Lagerung aus dem Spritzenkörper 70 herausfällt und der Inhalt der Fertigglasspritze 7 ausläuft.

Erfindungsgemäß wird daher vorgeschlagen, die Teilfläche 22 gemäß dem vorstehend beschriebenen erfindungsgemäßen Beschichtungsverfahren mit einer Atmosphärendruckplasmaquelle 101 und einer Unterdruckquelle 103 zu behandeln, welche erfindungsgemäß in Axialrichtung A bezüglich des Spritzenkonus 72 gegenüberliegend zueinander angeordnet sind und wobei die Unterdruckquelle 103 einen Unterdruck insbesondere in dem Spritzenkonus 72 kleiner als Atmosphärendruck bereitstellt, um die Adhäsionsfähigkeit von Klebstoff an der Teilfläche 22 der Innenfläche 21 der Fertigglasspritze 7 zu verstärken, damit eine Haltekraft zwischen Kanüle bzw. Injektionsnadel 73 und Glasspritze 7 gemäß DIN ISO 7864 erzielt wird. Durch die Änderung des Elementinventars und/oder der Bindungsverhältnisse der Konstituenten kann die hydrophobe Beschichtung 3 in der zweiten Teilfläche hydrophil werden, und so die Haftfestigkeit der Klebeverbindung 6 verbessern.

Nach dem Einkleben der Kanüle 76 in den Spritzenkörper 70 wird die Fertigglasspritze 7 in an sich bekannter Weise zur Befüllung vorbereitet, d.h. gereinigt, sterilisiert und verpackt. Hierbei wird auch das Nadelschutzteil 74 auf die Kanüle 76 aufgesetzt. Die auf diese Weise zur Befüllung vorbereitete Spritze wird dann an den Hersteller des Medikamentes zur Befüllung ausgeliefert.

Die in der vorstehenden Beschreibung, den Figuren und den Ansprüchen offenbarten Merkmale können sowohl einzeln als auch in beliebiger Kombination für die Realisierung der Erfindung in den verschiedenen Ausgestaltungen von Bedeutung sein.

### Bezugszeichenliste

- 2: Bauteil
- 3: Beschichtung
- 4: Plasma
- 6: Klebeverbindung
- 7: Fertigglasspritze
- 21: Innenfläche
- 22: Teilfläche
- 51: Gegenelektrode
- 52: Hochspannungselektrode
- 53: Isolator
- 54: Gaszufuhr
- 55: Hochspannungsquelle
- 70: Glasspritzenkörper
- 71: Kolben
- 72: Spritzenkonus
- 73: Injektionsnadel
- 74: Nadelschutzteil
- 75: Kolbenstopfen
- 76: Kanüle
- 100: Plasmabehandlungsvorrichtung
- 101: Atmosphärendruckplasmaquelle
- 103: Unterdruckquelle
- 105: Saugdorn
- 107: Kolbenraum
- 109: Innenseite
- 111: Bodenfläche
- 113: Kolbenrauminnendurchmesser
- 115: Einführlänge
- 119: Dichtring
- 123: Stirnende
- 155: Zwischenraum
- 157: Mündung
- 159: Austrittsöffnung
- 171: Flanschabschnitt
- 173: Flanschende
- 175: Kolbenabschnitt
- 177: Trichterabschnitt
- 179: Durchgang bzw. Endkanal
- 181: Spritzenkonus-Ende
- 183: Flanschsteg
- 185: Traggestell
- 187: Greifer
- 189: Fase
- 191, 193: Schlitz
- 197: Längsstrebe
- 199: Ständer
- 201: Sensoreinrichtung
- 203: Lasereinrichtung
- 205: Laserstrahlerzeuger
- 207: Reflexionseinrichtung
- 209: Laserstrahl

- A: Axialrichtung
- M: Mittelachse

## Patentansprüche

1. Verfahren zur Beschichtung eines Glasspritzenkörpers (70) für eine hypodermische Fertigglasspritze (7), bei welchem zumindest auf eine Innenfläche (21, 22) der hypodermischen Fertigglasspritze (7), die eine Axialrichtung (A) definiert, zumindest eine Emulsion und/oder eine Lösung aufgebracht wird, welche zumindest eine schichtbildende Substanz enthält, wobei zumindest eine Teilfläche (22) der Innenfläche (21) in einem Spritzenkonus (72) der Fertigglasspritze (7) nachfolgend einem Plasma (4) ausgesetzt wird, **dadurch gekennzeichnet, dass** relativ zu dem Spritzenkonus (72) in Axialrichtung (A) gegenüberliegend zu einer Athmosphärendruckplasmaquelle (101) eine Unterdruckquelle (103) angeordnet wird, wobei mittels der Unterdruckquelle (103) ein Unterdruck kleiner als Atmosphärendruck bereitgestellt wird.

2. Verfahren nach Anspruch 1, wobei ein Saugdorn (105) der Unterdruckquelle (103) in einen Kolbenraum (107) des Glasspritzenkörpers (70) eingeführt wird.

3. Verfahren nach Anspruch 2, wobei der Saugdorn (105) in einen vorzugsweise abdichtenden Berührkontakt mit einer Innenseite (109) und/oder einer Bodenfläche (111) des Kolbenraums (107) gebracht wird.

4. Verfahren nach einem der Ansprüche 1 bis 3, **dadurch gekennzeichnet, dass** der Kohlenstoffgehalt der Beschichtung (3) auf weniger als etwa 80% oder weniger als etwa 75% oder weniger als etwa 70% oder weniger als etwa 60% des Ausgangswertes vor der Plasmabehandlung abnimmt und/oder dass die Schichtdicke der Beschichtung (3) zumindest in der Teilfläche (22) der Innenfläche (21) vor der Einwirkung des Plasmas zwischen etwa 20 nm und etwa 100 nm oder zwischen etwa 30 nm und etwa 70 nm beträgt und durch die Plasmabehandlung in der Teilfläche (22) um mehr als etwa 20% oder mehr als etwa 25 % oder mehr als etwa 30 % abnimmt und/oder dass nach der Plasmabehandlung in der Teilfläche (22) eine Schichtdicke von mehr als etwa 70% oder mehr als etwa 60 % oder mehr als etwa 50 % verbleibt.

5. Verfahren nach einem der vorstehenden Ansprüche, **dadurch gekennzeichnet, dass** die Emulsion und/oder die Lösung zumindest ein Silikonöl und optional Wasser enthält oder daraus besteht und/oder
dass die Beschichtung (3) zumindest Kohlenstoff und Sauerstoff und Wasserstoff und
Silicium enthält und/oder
dass die Beschichtung (3) zumindest ein Poly(organo)siloxan enthält.

6. Verfahren nach einem der vorstehenden Ansprüche, **dadurch gekennzeichnet, dass** die Teilfläche (22) der Innenfläche (21) des Spritzenkonus (72) vor der Plasmabehandlung hydrophob und nach der Plasmabehandlung hydrophil ist.

7. Verfahren nach einem der vorstehenden Ansprüche, **dadurch gekennzeichnet, dass** dass das Plasma (4) ein Aktivgas enthält oder daraus besteht und
dass das Aktivgas optional Sauerstoff oder synthetische Luft oder atmosphärische Luft enthält oder daraus besteht.

8. Verfahren nach einem der vorstehenden Ansprüche, **dadurch gekennzeichnet, dass** das Plasma (4) für etwa 0,4 bis etwa 5 Sekunden oder für etwa 0,5 bis etwa 4 Sekunden oder für etwa 0,5 bis etwa 1,5 Sekunden oder für etwa 5 bis etwa 60 Sekunden einwirkt und/oder
dass ein Atmosphärendruckplasma zum Einsatz kommt, welches mit einer dielektrisch behinderten Entladung erzeugt wird und/oder
dass das Plasma (4) ein Inertgas enthält oder daraus besteht und
dass das Inertgas optional ein Edelgas, insbesondere Argon, enthält oder daraus besteht und/oder
dass das Plasma als Plasmastrahl oder Plasmajet ausgebildet ist, welcher zumindest auf die Teilfläche (22) der Innenfläche (21) des Spritzenkonus (72) einwirkt
und/oder das Plasma (4) einwirkt, während der Unterdruck bereitgestellt wird.

9. Verfahren zur Herstellung einer hypodermischen Fertigglasspritze (7) mit einem Glasspritzenkörper (70), der mit einem Verfahren nach einem der Ansprüche 1 bis 7 beschichtet wird, und einer Injektionsnadel (73 ), welche miteinander gefügt werden, wobei
die Injektionsnadel (73) entlang einer Fügestelle (6) mittels eines Klebstoffs mit dem Glasspritzenkörper (70) verbunden wird, wobei die Fügestelle (6) die Teilfläche (22) in dem Spritzenkonus (72) der Fertigglasspritze (7) umfasst.

10. Verfahren nach einem der Anspruch 9, **dadurch gekennzeichnet, dass** der Klebstoffes ausgewählt wird aus einem Acrylat und/oder einem Polyurethan und/oder einem Epoxidharz und/oder einem Cyanacrylat.

11. Plasmabehandlungsvorrichtung (100) für Glasspritzenkörper (70) hypodermischer Fertigglasspritzen (7), umfassend:
eine Athmosphärendruckplasmaquelle (101) zum Bereitstellen von Plasma zum Behandeln einer Innenfläche (21) eines Spritzenkonus (72) des Glasspritzenkörpers (70) und
eine Unterdruckquelle (103) zum Bereitstellen eines Unterdrucks kleiner als Athmosphärendruck, wobei die Athmosphärendruckplasmaquelle (101) und die Unterdruckquelle (103) relativ zu dem Spritzenkonus (72) einander gegenüberliegend anordenbar sind.

12. Plasmabehandlungsvorrichtung (100) nach Anspruch 11, wobei die Unterdruckquelle (103) einen Saugdorn (105) mit einem Außendurchmesser kleiner oder gleich einem Kolbenraum-Innendurchmesser (113) des Glasspritzenkörpers (70), insbesondere des Kolbenraums (107), aufweist.

13. Plasmabehandlungsvorrichtung (100) nach Anspruch 12, wobei der Saugdorn (105) eine Einführlänge (115) aufweist, entlang welcher der Außendurchmesser kleiner als der Kolbenraum-Innendurchmesser (113) des Glasspritzenkörpers (70) ist, wobei die Einführlänge (115) wenigstens so lang ist wie die Zylinderhöhe (117) des Kolbenraums (107), wobei insbesondere die Plasmabehandlungsvorrichtung (100) dazu eingerichtet ist, den Saugdorn (105) in den Kolbenraum (107) des Glasspritzenkörpers (70) vorzugsweise bis zu einem Berührkontakt mit einer Bodenfläche (111) des Kolbenraums (107) einzuführen.

14. Plasmabehandlungsvorrichtung (100) nach Anspruch 12 oder 13, wobei der Saugdorn (105) ein Dichtmittel, wie einen Dichtring (119) und/oder einen Dichtschlauch umfasst, das vorzugsweise zumindest am Stirnende (123) des Saugdorns (105) angeordnet ist, insbesondere um in einen vorzugsweise abdichtenden Berührkontakt mit einer Innenseite (109) und/oder einer Bodenfläche (111) des Kolbenraums (107) gebracht zu werden.

## Claims

1. A method for coating a glass syringe body (70) for a hypodermic pre-filled glass syringe (7), in which at least one emulsion and/or a solution containing at least one film-forming substance is applied to at least one inner surface (21, 22) of the hypodermic pre-filled glass syringe (7), which defines an axial direction (A), wherein at least one partial surface (22) of the inner surface (21) in a syringe cone (72) of the pre-filled glass syringe (7) is subsequently exposed to a plasma (4),
**characterized in that**
a vacuum source (103) is arranged relative to the syringe cone (72) in the axial direction (A) opposite to an atmospheric pressure plasma source (101), wherein a vacuum less than atmospheric pressure is provided by means of the vacuum source (103).

2. The method according to claim 1, wherein a suction mandrel (105) of the vacuum source (103) is introduced into a piston chamber (107) of the glass syringe body (70).

3. The method according to claim 2, wherein the suction mandrel (105) is brought into a preferably sealing contact with an inner side (109) and/or a bottom surface (111) of the piston chamber (107).

4. The method according to any one of claims 1 to 3, **characterized in that** the carbon content of the coating (3) decreases to less than about 80% or less than about 75% or less than about 70% or less than about 60% of the initial value before the plasma treatment and/or that the layer thickness of the coating (3) at least in the partial surface (22) of the inner surface (21) before the action of the plasma is between about 20 nm and about 100 nm or between about 30 nm and about 70 nm and decreases by more than about 20% or more than about 25% or more than about 30% due to the plasma treatment in the partial surface (22) and/or that after the plasma treatment in the partial surface (22) a layer thickness of more than about 70% or more than about 60% or more than about 50% remains.

5. The method according to any one of the preceding claims, **characterized in that** the emulsion and/or the solution contains or consists of at least a silicone oil and optionally water and/or
that the coating (3) contains at least carbon and oxygen and hydrogen and silicon and/or
that the coating (3) contains at least a poly(organo)siloxane.

6. The method according to any one of the preceding claims, **characterized in that** the partial surface (22) of the inner surface (21) of the syringe cone (72) is hydrophobic before the plasma treatment and hydrophilic after the plasma treatment.

7. The method according to any one of the preceding claims, **characterized in that** the plasma (4) contains or consists of an active gas and
that the active gas optionally contains or consists of oxygen or synthetic air or atmospheric air.

8. The method according to any one of the preceding claims, **characterized in that** the plasma (4) acts for about 0.4 to about 5 seconds or for about 0.5 to about 4 seconds or for about 0.5 to about 1.5 seconds or for about 5 to about 60 seconds and/or that an atmospheric pressure plasma is used, which is generated with a dielectric barrier discharge and/or
that the plasma (4) contains or consists of an inert gas and
that the inert gas optionally contains or consists of a noble gas, in particular argon, and/or
that the plasma is formed as a plasma beam or plasma jet, which acts at least on the partial surface (22) of the inner surface (21) of the syringe cone (72) and/or the plasma (4) acts while the vacuum is provided.

9. A method for manufacturing a hypodermic pre-filled glass syringe (7) with a glass syringe body (70), which is coated with a method according to any one of claims 1 to 7, and an injection needle (73), which are joined together,
wherein
the injection needle (73) is connected to the glass syringe body (70) along a joining point (6) by means of an adhesive, wherein the joining point (6) comprises the partial surface (22) in the syringe cone (72) of the pre-filled glass syringe (7).

10. The method according to claim 9, **characterized in that** the adhesive is selected from an acrylate and/or a polyurethane and/or an epoxy resin and/or a cyanoacrylate.

11. A plasma treatment device (100) for glass syringe bodies (70) of hypodermic pre-filled glass syringes (7), comprising:
an atmospheric pressure plasma source (101) for providing plasma for treating an inner surface (21) of a syringe cone (72) of the glass syringe body (70) and
a vacuum source (103) for providing a vacuum less than atmospheric pressure, wherein the atmospheric pressure plasma source (101) and the vacuum source (103) are arrangeable relative to the syringe cone (72) opposite each other.

12. The plasma treatment device (100) according to claim 11, wherein the vacuum source (103) has a suction mandrel (105) with an outer diameter less than or equal to an inner diameter of the piston chamber (113) of the glass syringe body (70), in particular of the piston chamber (107).

13. The plasma treatment device (100) according to claim 12, wherein the suction mandrel (105) has an insertion length (115) along which the outer diameter is less than the inner diameter of the piston chamber (113) of the glass syringe body (70), wherein the insertion length (115) is at least as long as the cylinder height (117) of the piston chamber (107), wherein in particular the plasma treatment device (100) is arranged to introduce the suction mandrel (105) into the piston chamber (107) of the glass syringe body (70) preferably up to a contact with a bottom surface (111) of the piston chamber (107).

14. The plasma treatment device (100) according to claim 12 or 13, wherein the suction mandrel (105) comprises a sealing means, such as a sealing ring (119) and/or a sealing hose, which is preferably arranged at least at the front end (123) of the suction mandrel (105), in particular to be brought into a preferably sealing contact with an inner side (109) and/or a bottom surface (111) of the piston chamber (107).

## Revendications

1. Procédé, destiné à revêtir un corps (70) de seringue en verre pour une seringue en verre (7) hypodermique prête à l'emploi, lors duquel l'on applique sur une surface intérieure (21, 22) de la seringue en verre (7) hypodermique prête à l'emploi, qui définit une direction axiale (A) au moins une émulsion et / ou une solution, laquelle contient au moins une substance filmogène, au moins une surface partielle (22) de la surface intérieure (21) étant exposée ensuite dans un cône de seringue (72) de la seringue en verre (7) hypodermique prête à l'emploi à un plasma (4), **caractérisé en ce que** dans la direction axiale (A) par rapport au cône de seringue (72), l'on place au vis-à-vis d'une source de plasma (101) sous pression atmosphérique une source de dépression (103), au moyen de la source de dépression (103) étant mise à disposition une dépression inférieure à la pression atmosphérique.

2. Procédé selon la revendication 1, lors duquel l'on introduit un mandrin aspirant (105) de la source de dépression (103) dans une chambre de piston (107) du corps (70) de la seringue en verre.

3. Procédé selon la revendication 2, lors duquel l'on amène le mandrin aspirant (105) dans un contact par effleurement assurant de préférence une étanchéité avec une face intérieure (109) et / ou une surface de fond (111) de la chambre de piston (107).

4. Procédé selon l'une quelconque des revendications 1 à 3, **caractérisé en ce que** la teneur en carbone du revêtement (3) décroît à moins d'environ 80 % ou à moins d'environ 75 % ou à moins d'environ 70 % ou à moins d'environ 60 % de la valeur initiale antérieure au traitement au plasma et / ou **en ce qu'**avant l'action du plasma, l'épaisseur de couche du revêtement (3) au moins dans la surface partielle (22) de la surface intérieure (21) s'élève à entre environ 20 nm et environ 100 nm ou à entre environ 30 nm et environ 70 nm et diminue du fait du traitement au plasma dans la surface partielle (22) de plus d'environ 20 % ou de plus d'environ 25 % ou de plus d'environ 30 % et / ou
**en ce qu'**après le traitement au plasma dans la surface partielle (22), il reste une épaisseur de couche de plus d'environ 70 % ou de plus d'environ 60 % ou de plus d'environ 50 %.

5. Procédé selon l'une quelconque des revendications précédentes, **caractérisé en ce que** l'émulsion et / ou la solution contient au moins une huile de silicone et optionnellement de l'eau ou est constituée de ceux-ci ou et / ou
**en ce que** le revêtement (3) contient au moins du carbone et de l'oxygène et de l'hydrogène et du silicium et / ou
**en ce que** le revêtement (3) contient au moins un poly(organo)siloxane.

6. Procédé selon l'une quelconque des revendications précédentes, **caractérisé en ce que** la surface partielle (22) de la surface intérieure (21) du cône de seringue (72) est hydrophobe avant le traitement au plasma et hydrophile après le traitement au plasma.

7. Procédé selon l'une quelconque des revendications précédentes, **caractérisé en ce que** le plasma (4) contient un gaz actif ou est constitué de celui-ci et **en ce que** le gaz actif contient optionnellement de l'oxygène ou de l'air synthétique ou de l'air atmosphérique ou est constitué de ceux-ci.

8. Procédé selon l'une quelconque des revendications précédentes, **caractérisé en ce que** le plasma (4) agit pour environ de 0,4 à environ 5 secondes ou pour environ de 0,5 à environ 4 secondes ou pour environ de 0,5 à environ 1,5 secondes ou pour environ de 5 à environ 60 secondes et / ou
**en ce que** l'on met en œuvre un plasma sous pression atmosphérique que l'on génère par une décharge rendue incomplète par voie diélectrique et / ou
**en ce que** le plasma (4) contient un gaz inerte ou est constitué de celui-ci et
**en ce que** le gaz inerte contient optionnellement un gaz rare, notamment de l'argon ou est constitué de celui-ci et / ou
**en ce que** le plasma est conçu sous la forme d'un faisceau de plasma ou d'un jet de plasma, lequel agit au moins sur la surface partielle (22) de la surface intérieure (21) du cône de seringue (72)
et / ou **en ce que** le plasma (4) agit pendant que la dépression est mise à disposition.

9. Procédé, destiné à fabriquer une seringue en verre (7) hypodermique prête à l'emploi avec un corps (70) d'une seringue en verre, que l'on revêt d'après un procédé selon l'une quelconque des revendications 1 à 7 et une aiguille d'injection (73), que l'on assemble l'une avec l'autre,
l'aiguille d'injection (73) étant reliée le long d'une zone de jointure (6) au moyen d'un agent adhésif avec le corps (70) d'une seringue en verre, la zone de jointure (6) comprenant la surface partielle (22) dans le cône de seringue (72) de la seringue (7) prête à l'emploi.

10. Procédé selon la revendication 9, **caractérisé en ce que** l'agent adhésif est sélectionné parmi un acrylate et / ou un polyuréthane et / ou une résine époxy et / ou un cyanoacrylate.

11. Dispositif (100) de traitement au plasma pour des corps (70) de seringues en verre de seringues (7), hypodermiques prêtes à l'emploi, comprenant :
une source de plasma (101) sous pression atmosphérique, destinée à mettre à disposition un plasma pour le traitement d'une surface intérieure (21) d'un cône de seringue (72) du corps (70) de la seringue en verre et
une source de dépression (103), destinée à mettre à disposition une dépression inférieure à la pression atmosphérique, la source de plasma (101) sous pression atmosphérique et la source de dépression (103) étant susceptibles d'être placées en vis-à-vis par rapport au cône de seringue (72).

12. Dispositif (100) de traitement au plasma selon la revendication 11, la source de dépression (103) comprenant un mandrin aspirant (105) d'un diamètre extérieur inférieur ou égal à un diamètre intérieur (113) de chambre du piston du corps (70) de la seringue en verre, notamment de la chambre de piston (107) .

13. Dispositif (100) de traitement au plasma selon la revendication 12, le mandrin aspirant (105) présentant une longueur d'introduction (115) le long de laquelle le diamètre extérieur est inférieur au diamètre intérieur (113) de chambre de piston du corps (70) de la seringue en verre, la longueur d'introduction (115) étant au moins aussi longue que la hauteur du cylindre (117) de la chambre de piston (107), notamment le dispositif (100) de traitement au plasma étant configuré pour introduire le mandrin aspirant (105) dans la chambre de piston (107) du corps (70) de la seringue en verre, de préférence jusqu'à un contact par effleurement avec une surface de fond (111) de la chambre de piston (107).

14. Dispositif (100) de traitement au plasma selon la revendication 12 ou 13, le mandrin aspirant (105) comprenant un moyen d'étanchéité, tel qu'une bague d'étanchéité (119) et / ou un flexible d'étanchéité qui est placé de préférence au moins sur l'extrémité frontale (123) du mandrin aspirant (105), notamment pour être amené dans un contact par effleurement assurant de préférence l'étanchéité avec une face intérieure (109) et / ou une surface de fond (111) de la chambre de piston (107).
